**Europäisches Patentamt**

**European Patent Office**

**Office européen des brevets**

(19)

(11) Veröffentlichungsnummer : **0 458 366 A2**

## (12) EUROPÄISCHE PATENTANMELDUNG

(21) Anmeldenummer : **91108530.6**

(22) Anmeldetag : **25.05.91**

(51) Int. Cl.$^5$ : **G07F 7/06, A61M 5/00**

(30) Priorität : **25.05.90 IT 352290**

(43) Veröffentlichungstag der Anmeldung :
**27.11.91 Patentblatt 91/48**

(84) Benannte Vertragsstaaten :
**AT BE CH DE DK ES FR GB LI NL**

(71) Anmelder : **Altei, Domenico**
**Via Mario Pellegrini 18**
**I-41058 Vignola (Modena) (IT)**
Anmelder : **Lapelosa, Marco**
**Via Francesco Petrarca 10**
**I-41013 Castelfranco Emilia (Modena) (IT)**

(72) Erfinder : **Altei, Domenico**
**Via Mario Pellegrini 18**
**I-41058 Vignola (Modena) (IT)**
Erfinder : **Lapelosa, Marco**
**Via Francesco Petrarca 10**
**I-41013 Castelfranco Emilia (Modena) (IT)**

(74) Vertreter : **Gustorf, Gerhard, Dipl.-Ing.**
**Patentanwalt Dipl.-Ing. Gerhard Gustorf**
**Bachstrasse 6 A**
**W-8300 Landshut (DE)**

(54) **Vorrichtung zum Handhaben von Spritzen.**

(57) Die Vorrichtung (1) zum Handhaben von Spritzen (3) hat eine Einheit (2) zum Erkennen von Spritzen (3). Diese Einheit weist einen rohrförmigen Körper (6) auf, der die Spritzen (3) nacheinander in im wesentlichen koaxialer Stellung aufnimmt. Zurückziehbare Halteelemente (8) dienen zum Fixieren der Spritzen (3). Auf den einander gegenüberliegenden Seiten der Halteelemente (8) sind zwei Erkennungselemente (12, 15) vorgesehen, die metallische Gegenstände erkennen und ein Signal zur Aktivierung einer Abgabeeinheit (4) für Gegenstände abgeben. Alternativ ist es auch möglich, daß der rohrförmige Körper (6) eine Querschnittsform hat, die im wesentlichen die Form der axialen Projektion einer Spritze (3) auf eine Ebene wiedergibt. Im Bereich von zwei Zonen, die die Form seitlich vorstehender Flanschabschnitte (26) der Spritzen (3) wiedergibt, sind zwei Sensoren (29) vorgesehen, die in Reihe hintereinander in eine Leitung (31) zur Abgabe eines Aktivierungssignals für die Abgabeeinheit (4) geschaltet sind.

EP 0 458 366 A2

# FIG.1

Die Erfindung betrifft eine Vorrichtung zum Handhaben von Spritzen und insbesondere eine solche Vorrichtung, die in der Lage ist, gebrauchte Spritzen aufzunehmen, als solche zu erkennen und nach diesem Erkennungsvorgang eine Abgabeeinheit zu betätigen, über welche Gegenstände wie, beispielsweise eine Münze oder eine neue Spritze ausgegeben werden.

Es ist bekannt, daß Drogenabhängige häufig gebrauchte Spritzen auf Straßen und Plätzen wegwerfen, nicht selten sogar nach wiederholtem Gebrauch und Weitergabe an andere Personen. Dies stellt eine erhebliche Gefahr für andere Menschen dar, die sich versehentlich mit der Nadel stechen, etwa beim Aufsammeln der Spritzen. Auf diese Weise können zahlreiche und schreckliche Krankheiten übertragen werden.

Überdies wird durch die Tatsache, daß die Spritzen mehrmals von verschiedenen Personen verwendet werden, die Ausbreitung von Krankheiten begünstigt.

Zur Lösung dieser Probleme ist bereits erwogen worden, gebrauchte Spritzen mit Hilfe von Apparaten zu sammeln, die in der Lage sind, für jede aufgenommene, gebrauchte Spritze eine neue Spritze oder eine Münze abzugeben, mittels der eine neue Spritze an einem Abgabeautomaten entnommen werden kann. Ein derartiger Apparat würde nicht zum Konsum von Rauschgiften ermuntern und die Süchtigen veranlassen, gebrauchte Spritzen nicht auf öffentlichen Straßen und Plätzen wegzuwerfen und regelmäßig neue Spritzen zu benutzen, wodurch die Ausbreitung der gefährlichen Krankheiten merklich eingeschränkt werden könnte. Dieses Projekt ist bisher jedoch lediglich im Absichtsstadium geblieben, und es ist nicht bekannt, daß derartige Apparate entwickelt oder gar ausgeführt worden sind.

Der Erfindung liegt die Aufgabe zugrunde, eine Vorrichtung zum Handhaben von Spritzen zu schaffen, die sehr einfach aufgebaut und zugleich äußerst zuverlässig ist, um eingeführte, gebrauchte Spritzen als solche zu erkennen und ggf. im Austausch einen Gegenstand abzugeben, beispielsweise eine Münze oder eine neue Spritze.

Gemäß der Erfindung wird diese Aufgabe bei einer Vorrichtung zum Handhaben von Spritzen dadurch gelöst, daß eine Erkennungseinheit vorgesehen ist, die die folgenden Komponenten hat: einen Rohrkörper zur Einzelaufnahme einer Spritze in einer zum Rohrkörper im wesentlichen koaxialen Stellung, Erkennungselemente zum Feststellen der Anwesenheit bestimmter Bereiche der Spritze und zur Abgabe eines Signals, wenn die Anwesenheit der bestimmten Abschnitte festgestellt worden ist, sowie Betätigungsorgane, die auf das Signal antworten und als Antwort darauf eine Abgabeeinheit betätigen, die mit der Erkennungseinheit gekoppelt ist.

Weitere Merkmale und Vorteile der Erfindung ergeben sich aus den Patentansprüchen, die Bestandteil dieser Beschreibung sind, sowie aus der folgenden Beschreibung von drei Ausführungsbeispielen, die in der Zeichnung dargestellt sind. Es zeigen:

Figur 1 eine schematische Schnittansicht einer ersten Ausführungsform der Vorrichtung gemäß der Erfindung,

Figur 2 in verkleinertem Maßstab eine Teilansicht der Vorrichtung der Figur 1 in einem anderen Arbeitszustand,

Figur 3 die Teilansicht einer abgeänderten Ausführungsform der Vorrichtung und

Figur 4 einen Querschnitt durch eine weitere Variante der Vorrichtung.

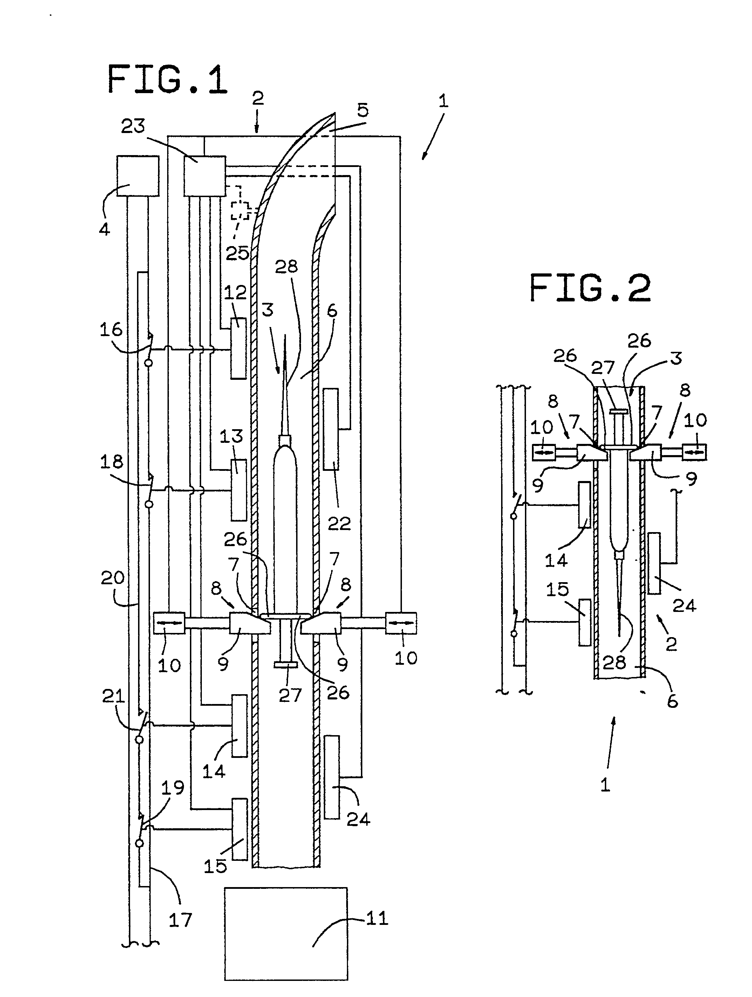

Die Figuren 1 und 2 zeigen eine Vorrichtung 1 zum Handhaben von Spritzen mit einer Erkennungseinheit 2 für gebrauchte Spritzen 3 sowie einer Arbeitseinheit, die eine Abgabeeinheit 4 aufweist, welche nach dem Erkennen einer gebrauchten Spritze 3 als solcher einen Gegenstand ausgibt, beispielsweise eine neue Spritze oder eine Münze.

Die Erkennungseinheit 2 hat einen Eingabestutzen 5, der zur Vertikalen geneigt ist und durch den die Spritzen 3 einzeln nacheinander eingeführt werden können. Der Eingabestutzen 5 führt an seinem unteren Ende zu einem vertikalen Rohr 6, dessen Durchmesser größer als der Durchmesser der Spritzen 3 ist und dessen Länge im wesentlichen der doppelten Länge einer Spritze 3 entspricht.

In seinem mittleren Bereich hat das Rohr 6 zwei diametral gegenüberliegende Öffnungen 7, durch welche zwei in Längsrichtung miteinander fluchtende und verschiebbare Halteelemente 8 vorstehen. Jedes Halteelement 8 hat einen horizontalen, im wesentlichen parallelflachen Finger 9, der an seiner Oberseite im Bereich des in das Rohr 6 reichenden, freien Endes abgeschrägt ist. Das außerhalb des Rohres 6 liegende Ende des Stabes 9 ist mit einem Betätigungsorgan 10 verbunden, mit dessen Hilfe der Finger 9 quer zum Rohr 6 verstellt werden kann.

Das untere Ende des Rohres 6 ist offen und befindet sich über einem Sammelbehälter 11 für Spritzen 3, die durch die Erkennungseinheit 2 hindurchgelaufen sind.

Entlang dem über den Fingern 9 liegenden Bereich des Rohres 6 befinden sich zwei Erkennungselemente 12 und 13 bekannter Bauart zum Erkennen der Anwesenheit von Metallen und sind so angeordnet, daß sie in der Höhe eines oberen bzw. eines unteren Abschnittes des genannten Bereiches des Rohres 6 liegen.

In ähnlicher Weise sind zwei Erkennungselemente 14 und 15 für Metall entlang dem Bereich des Rohres 6 vorgesehen, der unter den Fingern 9 liegt, und zwar in Höhe eines oberen bzw. eines unteren Abschnittes.

Wenn das Erkennungselement 12 einen metallischen Körper feststellt, gibt es ein elektrisches Signal ab, wodurch ein normalerweise geöffneter Schalter 16 geschlossen wird, der in eine elektrische Leitung 17 eingebaut ist, die mit der Abgabeeinheit 4 verbunden ist. Die Abgabeeinheit 4 ist mit der Erkennungseinheit 2 verbunden, welche vorzugsweise entsprechend der italienischen Patentanmeldung 3521 A/90 vom 25.05. 1990 ausgebildet ist. Die Abgabeeinheit 4 ist so ausgebildet, daß sie, wie bereits erwähnt, einen Gegenstand ausgibt, beispielsweise eine Münze oder eine Spritze, wenn sie von der Erkennungseinheit 2 hierfür die Zustimmung erhalten hat.

Wenn die Erkennungseinheit 13 keinen metallischen Körper feststellt, hält sie durch die Abgabe eines elektrischen Signals einen Schalter 18 geschlossen, der sich in der elektrischen Leitung 17 befindet und der von der Erkennungseinheit 13 dann geöffnet wird, wenn sich in ihrem Erkennungsbereich ein metallischer Gegenstand befindet.

Wenn das Erkennungselement 15 einen metallischen Körper feststellt, schließt es über die Abgabe eines elektrischen Signals einen normalerweise geöffneten Schalter 19, der sich in einer elektrischen Leitung 20 befindet, die parallel zur elektrischen Leitung 17 geschaltet ist. Das Erkennungselement 14 wirkt über ein elektrisches Signal auf einen Schalter 21 in der elektrischen Leitung 20 in derselben Weise wie das Erkennungselement 13 auf den Schalter 18.

Die elektrischen Leitungen 17 und 20 sind Leitungen zum Übertragen von Zustimmungssignalen für die Aktivierung der Abgabeeinheit 4 und können mit den Versorgungsleitungen für diese Abgabeeinheit 4 zusammenfallen.

Die genannten Schalter 16, 18, 19 und 21 werden nachstehend auch als Betätigungsglieder bezeichnet, da über sie und ihren paarweise geschlossenen Zustand die Betätigung der Abgabeeinheit 4 erfolgt.

Entlang einem mittleren Bereich des Rohres 6 oberhalb der Finger 9 ist ein Erkennungselement 22 zum Feststellen der Anwesenheit eines Gegenstandes, im Ausführungsbeispiel einer Spritze 3 angeordnet, das beispielsweise aus einem induktiven Sensor besteht und mit einem bekannten Zeitgeber 23 verbunden ist. Wenn das Erkennungselement 22 eine Spritze 3 festgestellt hat, aktiviert der Zeitgeber 23 nach einer vorbestimmten Zeit die Erkennungselemente 12, 13, 14 und 15, worauf nach einer bestimmten Zeit im Anschluß an die Aktivierung die Betätigungsorgane 10 eingeschaltet werden.

In gleicher Weise ist unterhalb der Finger 9 neben einem mittleren Bereich des Rohres 6 ein Erkennungselement 24 vorgesehen, das die Gegenwart eines Gegenstandes feststellt und mit der bereits genannten Zeitschaltung 23 verbunden ist, um nach einer vorbestimmten Zeit im Anschluß an die Feststellung der Gegenwart einer Spritze 3 die Erkennungselemente 12, 13, 14 und 15 zu aktivieren.

Selbstverständlich könnte die reine und einfache Erfassung der Gegenwart eines Gegenstandes mittels der Erkennungselemente 22 und 24 von jeder gleichwirkenden Vorrichtung erfolgen, beispielsweise durch eine Fotozelle 25, die in der Lage ist, über eine nicht gezeigte Öffnung in dem Rohr 6 den Durchgang eines Gegenstandes durch dieses Rohr 6 hindurch festzustellen.

Im Einsatz der Vorrichtung müssen die gebrauchten Spritzen 3 über den Eingabestutzen 5 in die Erkennungseinheit 2 eingeführt werden. Wenn die Spritzen 3 mit der Nadel nach hinten weisend eingeführt werden, ergibt sich die in Figur 1 gezeigte Situation, während Figur 2 den Fall zeigt, bei dem eine Spritze 3 mit der Nadel voraus eingeführt wird. Während eine Spritze 3 in die Erkennungseinheit 2 eingeführt wird, halten die Betätigungsorgane 10 die Finger 9 in ihrer vorgerückten Stellung, in der sie teilweise in das Rohr 6 eingreifen (vgl. Figuren 1 und 2).

Im Fall der Figur 1 legt sich daher die Spritze mit ihren beiden seitlich vorstehenden Flanschabschnitten 26 an den Fingern 9 an, wobei das Druckende 27 des Kolbens der Spritze unterhalb der beiden Finger 9 liegt. Die Anwesenheit der Spritze 3 wird durch das Erkennungselement 22 festgestellt, welches darauf den Zeitgeber 23 aktiviert, der nach einer vorbestimmten Zeit die Erkennungselemente 12 und 13 betätigt.

Wenn der in das Rohr 6 eingeführte Gegenstand tatsächlich eine Spritze 3 ist, stellt das Erkennungselement 12 die Anwesenheit der Nadel 28 fest und schließt dadurch den Schalter 16, während das Erkennungselement 13 den Kunststoffbereich der Spritze 3, d.h. die Abwesenheit metallischer Teile feststellt, wodurch der Schalter 18 geschlossen wird.

Das gleichzeitige Schließen der Schalter 16 und 18 erzeugt den durchgängig leitenden Zustand der elektrischen Leitung 17, so daß die Abgabeeinheit 4 betätigt wird. Nach der Betätigung der Abgabeeinheit 4 ziehen die Betätigungsorgane 10 die beiden Finger 9 zurück, so daß die im Rohr 6 befindliche Spritze 3 in den Sammelbehälter 11 fällt.

Wenn die Spritze 3 mit vorwärtsgerichteter Nadel 18 in das Rohr 6 eingeführt wird, wie Figur 2 zeigt, stützt sie sich mit ihren beiden seitlich vorstehenden Flanschabschnitten 26 ebenfalls an den Fingern 9 ab, während das Druckende 27 des Kolbens oberhalb dieser Finger 9 liegt. In dieser Position stellt das Erkennungselement 24 die Gegenwart der Spritze fest und aktiviert entsprechend den bereits erläuterten Abläufen den Zeitgeber 23, welcher nach einer vorbestimmten Zeit seinerseits die Erkennungselemente 14 und 15 aktiviert. Diese erkennen das Vorhandenseins des Körpers bzw. der Nadel 28 der Spritze 3, wodurch die Schalter 21 und 19 geschlossen werden und die nun nicht unterbrochene elektrische Leitung 20 die Weiterleitung eines Aktivierungssignals an die Abgabeeinheit 4 ermöglicht.

Die beiden Erkennungselemente 22 und 24 und der damit verbundene Zeitgeber 23 verhindern, daß die Erkennungseinheit 2 bereits dann unmittelbar aktiviert wird, wenn in das Rohr 6 ein Gegenstand eingeführt wird, der keine Spritze ist und von den Fingern 9 nicht in der Kontrollposition gehalten werden kann. Ein solcher Gegenstand fällt an den Fingern 9 vorbei sofort in den Sammelbehälter 11, bevor der Zeitgeber 23 die Erkennungselemente 12, 13, 14 und 15 aktivieren kann. Wenn die Erkennungselemente 12, 13, 14 und 15 aktiviert werden und dabei kein Gegenstand mehr in dem Rohr 6 ist, können diese in keiner Weise die Abgabeeinheit 4 in Gang setzen.

Wenn in das Rohr 6 Gegenstände eingeführt werden, die keine Spritzen, jedoch in der Lage sind, von den Fingern 9 angehalten zu werden, aktiviert das Erkennungselement 22 den Zeitgeber 23, der nach einer bestimmten Zeit die Erkennungselemente 12 und 13 aktiviert. Da es jedoch bei einem von einer Spritze verschiedenen Gegenstand sehr unwahrscheinlich ist, daß dieser einen oberen oder unteren, metallischen Abschnitt und einen verbleibenden nichtmetallischen Abschnitt hat, werden die beiden Erkennungselemente 12 und 13 normalerweise niemals in die Lage kommen, paarweise die Schalter 16 und 18 zu schließen, so daß die Abgabeeinheit 4 nicht betätigt wird. Nach einer bestimmten Zeit in Anschluß an den Kontrollvorgang mit negativem Ergebnis durch die Erkennungselemente 12 und 13 aktiviert der Zeitgeber 23 die Betätigungsorgane 10, wodurch die beiden Finger 9 zurückgezogen werden und der Gegenstand in den Behälter 11 fällt.

Die Erkennungseinheit 2 ist somit in der Lage, unabhängig von der Einführrichtung der Spritze 3 in das Rohr 6 fehlerfrei zu arbeiten, was eine zusätzliche Garantie für die Zuverlässigkeit der Erkennungseinheit 2 darstellt.

Über das beschriebene Ausführungsbeispiel der Vorrichtung 1 gemäß der Erfindung hinaus sind selbstverständlich Abänderungen im Rahmen des Erfindungsgedankens möglich.

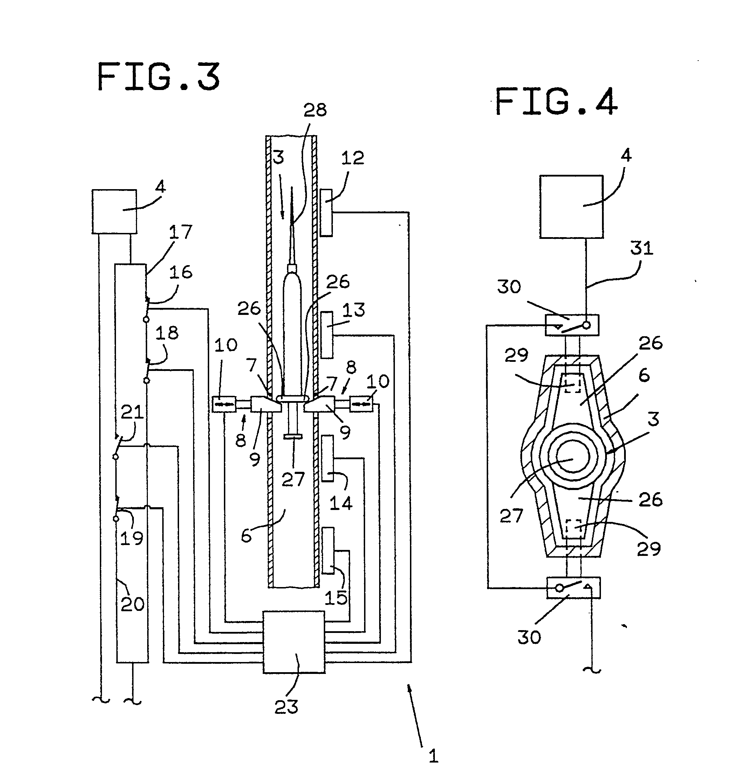

So können beispielsweise gemäß der schematischen Darstellung in Figur 3 die Erkennungselemente 22 und 24 fehlen, während die Erkennungselemente 12, 13, 14 und 15 auch als Mittel zum Erkennen der Anwesenheit von Gegenständen in dem Rohr 6 dienen, wobei sie mit der Zeitschaltung 23 und nicht mit den Schaltern 16, 18, 19 und 21 verbunden sind. Die Schalter 16, 18, 19 und 21 sowie die Betätigungsorgane 10 würden in diesem Fall ähnlich wie bereits erläutert durch den Zeitgeber 23 aktiviert werden, und zwar nach einem Zeitintervall, das durch die zuvor erläuterte Kontrolloperation bestimmt ist, welche an den Gegenständen durch die Erkennungselemente 12, 13, 14 und 15 vorgenommen worden ist.

Bei der in Figur 4 gezeigten Variante der Erkennungseinheit 2 hat der Innenumfang des Querschnittes des Rohres 6 zumindest in der Kontrollzone, in der die Spritzen 3 erkannt werden sollen, eine Form, die im wesentlichen die Form des Außenumfangs einer Axialprojektion einer Spritze 3 auf eine Ebene wiedergibt. In dieser Kontrollzone befinden sich im Bereich der beiden Zonen, die die Außenform der beiden seitlich abstehenden Flanschabschnitte 26 einer Spritze 3 wiedergeben, die entsprechenden Sensoren 29 von zwei Mikroschaltern 30, die hintereinandergeschaltet in einer Leitung 31 zur Abgabe eines elektrischen Signals für die Aktivierung der Abgabeeinheit 4 liegen.

Wenn eine Spritze 3 in das Rohr 6 eingeführt worden ist, befinden sich die seitlich abstehenden Flanschabschnitte 26 im Bereich der Sensoren 29, wodurch diese elastisch nach unten bewegt und die Mikroschalter 30 geschlossen werden, was die Aktivierung der Abgabeeinheit 4 zur Folge hat.

Nachdem es sehr unwahrscheinlich ist, daß ein Gegenstand, der keine Spritze ist, die Querschnittsform so genau nachbildet, daß er nach dem Einführen in das Rohr 6 die Sensoren 29 betätigen kann, wird ein irgendwie geformter Gegenstand nach dem Einführen in das Rohr 6 normalerweise den Behälter 11 erreichen, ohne die Abgabeeinheit 4 aktiviert zu haben.

Gemäß einer nicht gezeigten Variante der Erkennungseinheit 2 kann der Bereich des Rohres 6 unterhalb der Finger 9 fehlen, wobei die in der Stellung der Figur 2 eingeführten Spritzen 3 während der Erkennungsoperation frei von den Fingern 9 nach unten herabhängen, um im Anschluß an die Erkennungsoperation in den Behälter 11 zu fallen.

Die Halteelemente 8 können in jeder geeigneten Weise ausgeführt werden, sofern sie nur in der Lage sind, während der Erkennungsoperation der Spritzen 3 diese in der zuvor erläuterten Stellung zu halten. Es ist auch möglich, daß die Halteelemente 8 andere Teile der Spritzen 3 als die seitlich abstehenden Flanschabschnitte 26 halten, wobei dann die notwendigen Änderungen an der Erkennungseinheit 2 lediglich darin bestehen, die Erkennungselemente 12, 13, 14 und 15 an entsprechend anderer Stelle anzuordnen.

Weiterhin können die Erkennungselemente 13 und 14 mit den zugehörigen Schaltern 18 und 21 fehlen. In diesem Fall wäre die Funktion der Erkennungseinheit 2 darauf beschränkt, einen in das Rohr 6 eingeführten Gegenstand daraufhin zu überprüfen, ob er in einem bestimmten Bereich des Rohres 6 einen metallischen Teil, vorzugsweise die Nadel einer Spritze, aufweist, wobei dieser bestimmte Teil in der Höhe liegen soll, in der normalerweise der metallische Teil angeordnet ist, um sicherzustellen, daß es sich bei dem Gegenstand tatsächlich um eine Spritze handelt.

Aus den obigen Ausführungen ergibt sich, daß die Vorrichtung 1 gemäß der Erfindung die gesteckten Ziele vollkommen erreicht. In der Praxis hat sich diese Vorrichtung als äußerst zuverlässig und trotzdem sehr einfach herausgestellt, so daß das hier zugrundeliegende Problem gelöst ist.

**Patentansprüche**

1. Vorrichtung zum Handhaben von Spritzen, gekennzeichnet durch eine Erkennungseinheit (2) für Spritzen (3), welche umfaßt einen rohrförmigen Körper (6) zur Aufnahme jeweils einer Spritze (3) in einer im wesentlichen koaxialen Stellung, Organe (12, 15, 13, 14,29) zum Erkennen der Anwesenheit bestimmter Bereiche der Spritze (3) und zur Abgabe eines Signals beim Erkennen der Anwesenheit der vorbestimmten Bereiche sowie Betätigungsglieder (16,19, 18,21,30) zur Abgabe einer Antwort auf dieses Signal und zur Zustimmung für die Aktivierung einer Arbeitseinheit (4), die mit der Erkennungseinheit (2) gekoppelt ist.

2. Vorrichtung nach Anspruch 1, dadurch gekennzeichnet, daß die Erkennungseinheit (2) zurückziehbare Elemente (8) zum Halten der Spritzen (3) aufweist, die in einem vorbestimmten Bereich des rohrförmigen Körpers (6) liegen, und daß die Erkennungsorgane zwei erste Erkennungselemente (12,15) zum Feststellen der Anwesenheit metallischer Gegenstände aufweisen, die in Längsrichtung des rohrförmigen Körpers (6) auf gegenüberliegenden Seiten der Halteelemente (8) liegen und zur Abgabe eines Signals beim Erkennen metallischer Gegenstände ausgelegt sind.

3. Vorrichtung nach Anspruch 2, dadurch gekennzeichnet, daß die Erkennungseinheit (2) zwei weitere Erkennungselemente (13,14) für metallische Gegenstände aufweist, die auf gegenüberliegenden Seiten der Halteelemente (8) und zwischen diesen und den ersten Erkennungselementen (12,15) liegen und zur Abgabe eines Signals beim Erkennen der Abwesenheit metallischer Gegenstände in ihrem Aktionsbereich ausgelegt sind, wobei Betätigungsglieder (18,21) zur Aufnahme dieses Signals und als Antwort darauf zur Zustimmung der Aktivierung der Arbeitseinheit (4) vorgesehen sind.

4. Vorrichtung nach Anspruch 2 oder 3, dadurch gekennzeichnet, daß die Erkennungseinheit (2) einen Zeitgeber (23) aufweist, der mit den Erkennungselementen (12,13,14,15) verbunden ist.

5. Vorrichtung nach Anspruch 4, dadurch gekennzeichnet, daß der Zeitgeber (23) mit den Betätigungsgliedern (16,18,19,21) verbunden ist.

6. Vorrichtung nach einem der vorhergehenden Ansprüche, dadurch gekennzeichnet, daß die Erkennungseinheit (2) zwei Erkennungselemente (22,24) zum Feststellen der Anwesenheit von Gegenständen in dem rohrförmigen Körper (6) aufweist, die in Längsrichtung des rohrförmigen Körpers (6) auf gegenüberliegenden Seiten der Halteelemente (8) angeordnet sind.

7. Vorrichtung nach den Ansprüchen 4 und 6, dadurch gekennzeichnet, daß der Zeitgeber (23) auch mit den Erkennungselementen (22,24) verbunden ist.

8. Vorrichtung nach Anspruch 1, dadurch gekennzeichnet, daß der Innenumfang des Querschnitts wenigstens eines Teiles des rohrförmigen Körpers (6) eine Form hat, die wenigstens im Bereich einer Kontrollzone, in der die Spritzen (3) für den Erkennungsvorgang positioniert werden sollen, im wesentlichen die Form des Außenumfangs einer Axialprojektion einer Spritze (3) auf eine Ebene wiedergibt, wobei die Organe zum Erkennen vorbestimmter Bereiche der Spritze Sensoren (29) aufweisen, die wenigstens einen seitlich vorstehenden Flanschabschnitt (26) der Spritze (3) erkennen.

9. Vorrichtung nach Anspruch 8, dadurch gekennzeichnet, daß die Sensoren (29) mit wenigstens einem Schalter (30) verbunden sind, der in einer Leitung (31) zur Abgabe eines Aktivierungssignals für die Arbeitseinheit (4) liegt.

10. Vorrichtung nach Anspruch 8 oder 9, dadurch gekennzeichnet, daß die Sensoren (29) so ausgelegt sind, daß sie die beiden seitlich vorstehenden Flanschabschnitte (26) der Spritze (3) erkennen und mit zwei Schaltern (30) verbunden sind, die in Reihe hintereinander in die Leitung (31) zur Abgabe eines Aktivierungssignals für die Arbeitseinheit (4) geschaltet sind.

11. Vorrichtung nach einem der vorhergehenden Ansprüche, dadurch gekennzeichnet, daß die Arbeitseinheit aus einer Abgabeeinheit (4) zur Abgabe von Gegenständen besteht, die mit der Erkennungseinheit (2) zum Erkennen der Spritzen (3) verbunden ist.

12. Vorrichtung nach Anspruch 11, dadurch gekennzeichnet, daß die Gegenstände neue Spritzen sind.

13. Vorrichtung nach Anspruch 11, dadurch gekennzeichnet, daß die Gegenstände aus Münzen bestehen, die die Entnahme neuer Spritzen bei automatischen Spritzenabgabegeräten gestatten.

# FIG.1

# FIG.2

# FIG.3

# FIG.4